# EUROPEAN PATENT SPECIFICATION

(11) **EP 3 463 040 B1**
(45) Date of publication and mention of the grant of the patent: **26.05.2021**
(21) Application number: 17803188.6
(22) Date of filing: 22.02.2017
(51) Int. Cl.: A61B 1/012, A61B 1/015, A61B 1/12, A61B 1/00, A61B 1/06, A61B 1/018

(54) **MULTI-JET CONTROLLER FOR AN ENDOSCOPE**
MULTISTRAHLSTEUERGERÄT FÜR EIN ENDOSKOP
UNITÉ DE COMMANDE DE JETS MULTIPLES POUR UN ENDOSCOPE

(30) Priority: 23.05.2016 US 201662340121 P
(43) Date of publication of application: 10.04.2019
(73) Proprietor: Endochoice, Inc., Alpharetta, GA 30009 (US)
(72) Inventor: AIZENFELD, Amram, 1924500 Ramot Menashe (IL); SALMAN, Golan, 3030000 Atlit (IL); SCHWARCZ, Hadar, 4252458 Netanya (IL)
(74) Representative: Vossius & Partner Patentanwälte Rechtsanwälte mbB
(86) International application number: PCT/US2017/018972
(87) International publication number: WO 2017/204873

(56) References cited:
- WO-A1-2009/095915
- US-A1- 2006 106 285
- US-A1- 2006 276 689
- US-A1- 2011 224 654
- US-A1- 2012 088 974
- US-A1- 2015 005 581
- US-B1- 6 309 347
- US-B1- 8 881 752

## Description

### FIELD

The present specification generally relates to an endoscope assembly comprising a front jet and at least one side jet being supplied with fluid via fluid channels and a system to control direction of flow of fluid through the jets.

### BACKGROUND

Endoscopes provide a means for performing medical procedures with minimal patient trauma, while enabling the physician to view the internal anatomy of the patient. Over the years, numerous endoscopes have been developed and categorized according to specific applications, such as cystoscopy, colonoscopy, laparoscopy, upper GI endoscopy and others. Endoscopes may be inserted into the body's natural orifices or through an incision in the skin.

An endoscope is usually an elongated tubular shaft, rigid or flexible, having a video camera or a fiber optic lens assembly at its distal end. The shaft is connected to a handle, which sometimes includes an ocular element to enable direct viewing of the patient's anatomy. Various surgical tools may be inserted through a working channel in the endoscope for performing different surgical procedures.

Endoscopes, such as colonoscopes, gastroscopes and the like, that are currently being used, typically have a front camera for viewing internal organs, such as the colon, an illuminator, a fluid injector for cleaning the camera lens, and a working channel for inserting surgical tools in order to, for example, remove polyps found in the colon. Often, endoscopes also have fluid ("jet") injectors for cleaning a body cavity, such as the colon, into which they are inserted.

There is a need in the art for endoscopes which enable the concurrent, and multidirectional, supply of fluids to multiple fluid injectors or jet openings in the endoscope tip in order to quickly and efficiently clean a body cavity or a portion of the endoscope.

US 6,309,347 B1 discloses a system for endoscopes which can supply air and water according to each application by regulating air and water flow rates independently. Although air and water may be supplied with one pump, air and water flow rates are controlled separately by using an air supply pump and water supply pump and by controlling a plurality of open-close valves installed, for example, on atmospheric escape pipes. The air channel and water channel are connected, for draining, by a first connecting pipe.

US 2006/0106285 A1 discloses a fluid delivery system for use with an endoscope. Certain embodiments include a single, large fluid source and pump installed upon an operator console, in combination with a small fluid reservoir and pump installed within a proximal connector of the imaging endoscope, multiple fluid sources that feed a common fluid channel that are pressurized by a common pump, multiple fluid sources that feed dedicated fluid channels that are pressurized by dedicated pumps, and a small fluid reservoir and pump installed within a handheld manual controller of the imaging endoscope.

US 2015/0005581 A1 discloses a jet distributor provided to supply fluids to each of a plurality of jet openings in a multi jet endoscope tip. In an embodiment, the jet distributor is supplied with a fluid from a jet pump. The jet distributor includes at least two fluid channels to provide the fluid supplied via the jet pump to the front-jet, right-side-jets and left-side-jets in the endoscope tip. The jet-distributor includes a motor which rotates a rotating plug. The rotating plug includes an internal fluid pathway which becomes intermittently aligned with each of a plurality of fluid output channels in the jet distributor as the plug rotates, thereby providing fluid in a successive manner to the jets in the endoscope tip.

### SUMMARY

The following embodiments and aspects thereof are described and illustrated in conjunction with systems, tools and methods, which are meant to be exemplary and illustrative, not limiting in scope. The invention is defined in the independent claims. Embodiments are recited in the dependent claims.

The present specification discloses an apparatus for controlling a flow direction of fluid from a fluid source external to an endoscope into a plurality of fluid channels positioned within a distal end of the endoscope, comprising: a tube comprising a first combination of fluid channels and a second combination of fluid channels; a pump connected to the tube, wherein the pump is adapted to direct fluid from the external source to at least one of a first combination of fluid channels and a second combination of fluid channels in said tube; and a controller to activate the pump, wherein the controller is configured to cause the pump to direct fluid to the first combination of fluid channels upon a first activation of the controller and wherein the controller is configured to cause the pump to direct fluid to the second combination of fluid channels upon a second activation of the controller.

Optionally, the pump is a peristaltic pump.

Optionally, the apparatus of further comprises a user trigger to control said controller, wherein the user trigger comprises a button and wherein the button is configured such that pressing the button causes the first activation of the controller and pressing the button twice causes the second activation of the controller.

Optionally, the apparatus further comprises a user trigger to control said controller, wherein the user trigger comprises a lever and wherein the lever is configured such that pulling the lever causes the first activation of the controller and pushing the lever causes the second activation of the controller.

Optionally, the apparatus further comprises a user trigger to control said controller, wherein the user trigger comprises a pedal and wherein the pedal is configured such that stepping on the pedal causes the first activation of the controller and stepping on the pedal twice causes the second activation of the controller.

Optionally, the first combination of fluid channels and the second combination of fluid channels are co-linearly placed within the tube.

The first combination of fluid channels comprises a fluid channel that opens through a front jet in a distal tip of the endoscope.

The second combination of fluid channels may comprise a fluid channel that opens through a front jet in a distal tip of the endoscope and at least one side jet in the distal tip of the endoscope.

Optionally, the pump is configured to direct fluid in a first direction through the first combination of fluid channels upon said first activation of the controller, the pump is configured to direct fluid in a second direction through the second combination of fluid channels upon said second activation of the controller, and the first direction is different from the second direction.

Optionally, the apparatus further comprises a first check valve in a first fluid channel positioned between said external source and the pump and a second check valve in a second fluid channel positioned between said external source and the pump, wherein the first fluid channel is separate from the second fluid channel. Optionally, the first fluid channel is in fluid communication with the first combination of fluid channels, the second fluid channel is in fluid communication with the second combination of fluid channels, the first fluid channel is not in fluid communication with the second combination of fluid channels, and the second fluid channel is not in fluid communication with the first combination of fluid channels.

The present specification also discloses a method for controlling a flow direction of fluid from a fluid source external to an endoscope into a plurality of fluid channels positioned within the endoscope, comprising: receiving a user input into a trigger; based upon said trigger, using a controller to activate a pump connected to a tube, wherein said tube comprises a first combination of fluid channels and a second combination of fluid channels, wherein, upon a first activation of the pump, said pump causes fluid to flow in a first direction from the external source to the first combination of fluid channels, and wherein, upon a second activation of the pump, said pump causes fluid to flow in a second direction from the external source to the second combination of fluid channels.

Optionally, the pump comprises a peristaltic pump.

Optionally, said trigger comprises a button, wherein pressing the button once enables said first activation of the pump, and wherein pressing the button twice enables the second activation of the pump.

Optionally, said trigger comprises a lever, wherein pulling the lever enables the first activation of the pump and wherein pushing the lever enables the second activation of the pump.

Optionally, said trigger comprises a lever, wherein pulling or pushing the lever once enables the first activation of the pump and wherein pushing or pulling the lever twice enables the second activation of the pump.

Optionally, said trigger comprises a pedal, wherein pushing the pedal once causes the first activation of the pump and wherein pushing the pedal twice causes the second activation of the pump.

Optionally, the first combination of fluid channels and the second combination of fluid channels are co-linearly placed within the tube.

The first combination of fluid channels comprises a fluid channel that opens through a front jet in a distal tip of the endoscope.

The second combination of fluid channels may comprise a fluid channel that opens through a front jet in a distal tip of the endoscope and at least one side jet in the distal tip of the endoscope.

Optionally, the pump is configured to direct fluid in a first direction through the first combination of fluid channels upon said first activation of the pump, the pump is configured to direct fluid in a second direction through the second combination of fluid channels upon said second activation of the pump, and the first direction is different from the second direction.

Optionally, a first check valve is included in a first fluid channel positioned between said external source and the pump and a second check valve is included in a second fluid channel positioned between said external source and the pump, wherein the first fluid channel is separate from the second fluid channel. Optionally, the first fluid channel is in fluid communication with the first combination of fluid channels, the second fluid channel is in fluid communication with the second combination of fluid channels, the first fluid channel is not in fluid communication with the second combination of fluid channels, and the second fluid channel is not in fluid communication with the first combination of fluid channels.

The present specification also discloses a system for controlling a flow direction of fluid from a source external to an endoscope into a plurality of fluid channels positioned within the endoscope, comprising: a pump, wherein the pump is adapted to direct fluid from the external source to at least a first combination of fluid channels and a second combination of fluid channels; an activation system to activate the pump, wherein the pump directs fluid to the first combination of fluid channels upon a first activation by the activation system and the pump directs fluid to the second combination of fluid channels upon a second activation by the activation system; and at least one check valve connected in the plurality of fluid channels to control the flow of fluid during the first activation and the second activation.

Optionally, the activation system comprises a button, wherein the first activation comprises pressing the button once and the second activation comprises pressing the button twice.

Optionally, the activation system comprises a lever, wherein the first activation comprises pulling the lever and the second activation comprises pushing the lever.

Optionally, the activation system comprises a pedal, wherein the first activation comprises pushing the pedal once and the second activation comprises pushing the pedal twice.

Optionally, the pump is a peristaltic pump.

Optionally, the first combination of fluid channels comprises a fluid channel that opens through a front jet in a distal tip of the endoscope. Optionally, the second combination of fluid channels comprises a fluid channel that opens through a front jet in a distal tip of the endoscope and at least one side jet in the distal tip of the endoscope.

Optionally, the system further comprises at least one endoscope connector housing the plurality of endoscope fluid channels. Optionally, the at least one endoscope connector is positioned within the endoscope. Optionally, the at least one endoscope connector is positioned within a main control unit external to the endoscope.

The aforementioned and other embodiments of the present specification shall be described in greater depth in the drawings and detailed description provided below.

The invention is defined in the independent claims with the dependent claims defining preferred embodiments thereof.

### BRIEF DESCRIPTION OF THE DRAWINGS

These and other features and advantages of the present invention will be appreciated, as they become better understood by reference to the following detailed description when considered in connection with the accompanying drawings, wherein:
Figure 1 illustrates an exploded view of a tip section of an endoscope assembly according to one embodiment of the present specification;
Figure 2A illustrates a perspective view of a tip section of an endoscope assembly according to one embodiment of the present specification;
Figure 2B illustrates another perspective view of a tip section of an endoscope assembly according to one embodiment of the present specification;
Figure 3A illustrates a perspective view of a tip section of a multi-jet endoscope assembly according to one embodiment of the present specification;
Figure 3B illustrates a perspective first side view of the tip section of the multi-jet endoscope assembly of Figure 3A;
Figure 3C illustrates a perspective second side view of the tip section of the multi-jet endoscope assembly of Figure 3A;
Figure 3D illustrates a perspective view of a fluid channeling component of the multi-jet endoscope assembly of Figure 3A;
Figure 4 illustrates an exemplary main connector, which is known in the prior art;
Figure 5A illustrates an embodiment where a dual-direction jet pump enables fluid flow through a single pipeline tube to a main connector, in accordance with an embodiment of the present specification;
Figure 5B illustrates an exemplary coupling system, in accordance with an embodiment of the present specification;
Figure 5C is a block diagram illustrating connection between a dual-direction jet pump and an endoscope, in accordance with an embodiment of the present specification;
Figure 5D illustrates an exemplary operation of dual direction pump, in accordance with an embodiment of the present specification; and
Figure 6 is a flow chart illustrating an exemplary method of controlling a flow direction of fluid from a source external to an endoscope into a plurality of fluid channels positioned within the endoscope, in accordance with an embodiment of the present specification.

### DETAILED DESCRIPTION

In an embodiment, a pump is provided with an endoscope to control the flow of fluid from an external source to a combination of fluid channels within the endoscope, which supply fluid to a front jet or to the front jet and at least one side jet of a multi-jet endoscope assembly. In embodiments, the pump is a dual-direction pump that enables control of fluid to either the front jet or to the front and side jets of the endoscope. In various embodiments, the fluid flow control system of the present specification is intended for operation with a multiple viewing elements endoscope similar to those described in US 2014/0296866 A1, entitled "Multiple Viewing Elements Endoscope Having Two Front Service Channels" and filed on May 15, 2014.

The present specification is directed towards multiple embodiments. The following disclosure is provided in order to enable a person having ordinary skill in the art to practice the invention. Language used in this specification should not be interpreted as a general disavowal of any one specific embodiment or used to limit the claims beyond the meaning of the terms used therein. Also, the terminology and phraseology used is for the purpose of describing exemplary embodiments and should not be considered limiting. Thus, the present invention is to be accorded the widest scope encompassing numerous alternatives, modifications and equivalents consistent with the principles and features disclosed. For purpose of clarity, details relating to technical material that is known in the technical fields related to the invention have not been described in detail so as not to unnecessarily obscure the present invention. In the description and claims of the application, each of the words "comprise" "include" and "have", and forms thereof, are not necessarily limited to members in a list with which the words may be associated.

It should be noted herein that any feature or component described in association with a specific embodiment may be used and implemented with any other embodiment unless clearly indicated otherwise.

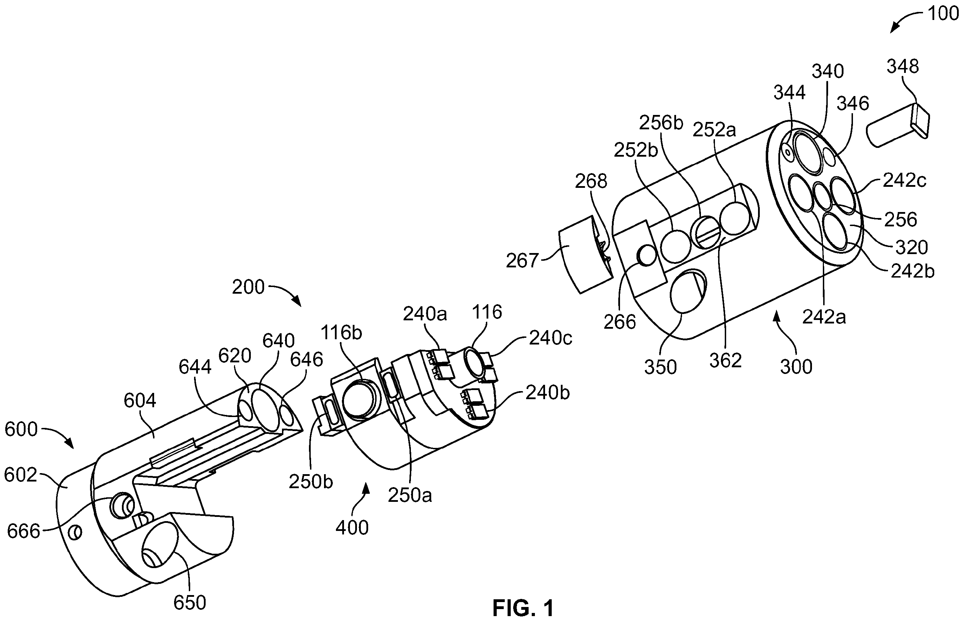

Reference is now made to Figure 1, which shows an exploded view of a tip section 200 of a multi-viewing element endoscope assembly 100 comprising at least one front working/service channel, according to various embodiments. An aspect of some embodiments also relates to endoscope assembly 100 having the tip section 200 equipped with one or more side working/service channels.

It is noted that the term "endoscope" as mentioned to herein may refer particularly to a colonoscope or gastroscope, according to some embodiments, but is not limited only to colonoscopes and gastroscopes. The term "endoscope" may refer to any instrument used to examine the interior of a hollow organ or cavity of the body.

According to an embodiment, tip section 200 of endoscope 100 includes a tip cover 300, an electronic circuit board assembly 400 and a fluid channeling component 600.

According to some embodiments, fluid channeling component 600 may be configured as a separate component from electronic circuit board assembly 400. This configuration may be adapted to separate the fluid channels, at least one side service channel, such as side service channel 650, and at least one front working/service channel, such as working/service channel 640, which are located in fluid channeling component 600, from the sensitive electronic and optical parts which may be located in the area of electronic circuit board assembly 400. Thus, the component structure of the tip section 200 enables effective insulation of the plurality of electronic elements from the plurality of fluid channels.

Tip section 200 may be turnable by way of flexible shaft which is also referred to as a bending section, for example a vertebra mechanism.

In some embodiments, electronic circuit board assembly 400 is configured to carry a front viewing element 116 and at least one side viewing element 116b which may be similar to front viewing element 116 and may include a sensor such as but not limited to a Charge Coupled Device (CCD) or a Complementary Metal Oxide Semiconductor (CMOS) image sensor. In addition, electronic circuit board assembly 400 may be configured to carry a second side viewing element on the opposite side of side viewing element 116b, which may be similar to front viewing element 116 and may include a sensor such as but not limited to a Charge Coupled Device (CCD) or a Complementary Metal Oxide Semiconductor (CMOS) image sensor.

Electronic circuit board assembly 400 may further be configured to carry front illuminators 240a, 240b, 240c, which are, in one embodiment, associated with front viewing element 116 and are positioned to essentially illuminate the fields of view of front viewing element 116.

In addition, electronic circuit board assembly 400 may further be configured to carry side illuminators 250a and 250b, which are, in one embodiment, associated with side viewing element 116b and are positioned to essentially illuminate the fields of view of side viewing element 116b. Electronic circuit board assembly 400 may also be configured to carry side illuminators, which are associated with a second side viewing element that is positioned on the opposite side of side viewing element 116b, which may be similar to side illuminators 250a and 250b.

Front illuminators 240a, 240b, 240c and side illuminators 250a and 250b may optionally be discrete illuminators and may include a light-emitting diode (LED), which may be a white light LED, an infrared light LED, a near infrared light LED, an ultraviolet light LED or any other LED.

The term "discrete", concerning discrete illuminator, may refer to an illumination source, which generates light internally-in contrast to a non-discrete illuminator, which may be, for example, a fiber optic merely transmitting light generated remotely.

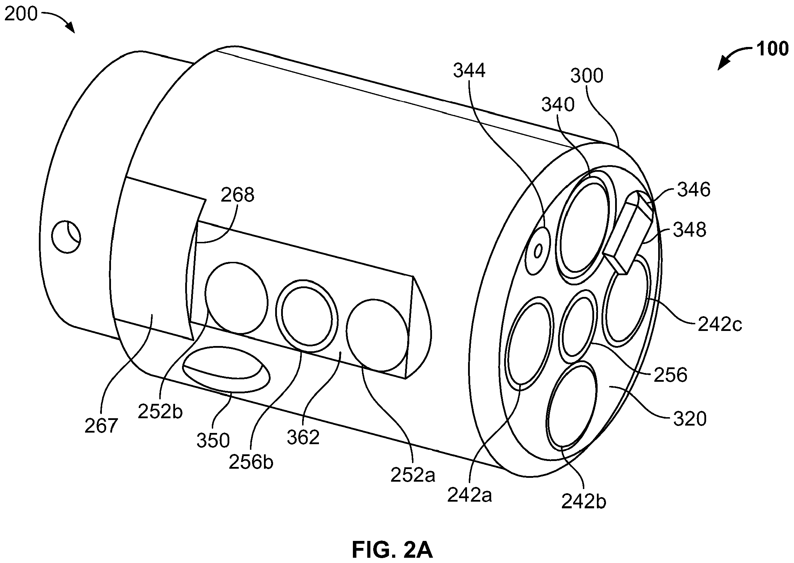

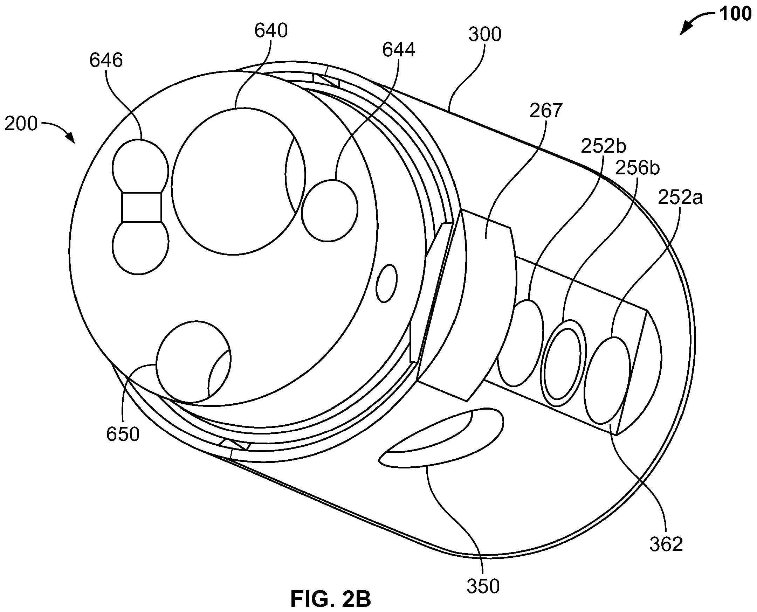

Reference is now made to Figure 1 along with Figure 2A and Figure 2B, which show a perspective view of a tip section 200 of an endoscope assembly 100 according to an embodiment.

Tip cover 300 may be configured to fit over the inner parts of the tip section 200 including electronic circuit board assembly 400 and fluid channeling component 600 and to provide protection to the internal components in the inner parts.

Tip cover 300 may include a front panel 320 having a transparent surface, window, or opening for front optical lens assembly 256, of front looking camera or viewing element 116. Front optical lens assembly 256 may include a plurality of lenses, static or movable, which may provide a field of view of 90 degrees or more, 120 degrees or more or up to essentially 180 degrees. Front optical lens assembly 256 may provide a focal length in the range of about 3 to 100 millimeters.

An optical axis of front looking camera or viewing element 116 may be essentially directed along the long dimension of the endoscope. However, since front viewing element 116 is typically a wide angle viewing element, its field of view may include viewing directions at large angles to its optical axis. Additionally, front panel 320 may include optical windows 242a, 242b and 242c of illuminators 240a, 240b and 240c, respectively. It should be noted that number of illumination sources used for illumination of the field of view may vary.

In addition, front panel 320 may include a working channel opening 340 of a working channel 640. In alternate embodiments, the front panel may include more than one working channel opening.

Jet channel opening 344 of jet channel 644 may also be located on front panel 320 of tip cover 300. Jet channel 644 may be configured for providing high-pressure jet of fluid such as water or saline for cleaning the walls of the body cavity.

Also located on front panel 320 of tip cover 300 is injector opening 346 of injector channel 646 having a nozzle 348 aimed at front optical lens assembly 256. Injector channel 646 may be configured for injecting fluid (liquid and/or gas) to wash contaminants such as blood, feces and other debris from a surface of front optical lens assembly 256 of front viewing element 116. Optionally, injector channel 646 may be configured for cleaning front optical lens assembly 256 and one, two, or all of optical windows 242a, 242b and 242c. Injector channel 646 may be fed by fluid such as water and/or gas which may be used for cleaning and/or inflating a body cavity.

Visible on the sidewall 362 of tip cover 300 is side optical lens assembly 256b for side viewing element 116b, which may be similar to front optical lens assembly 256 and optical windows 252a and 252b of illuminators 250a and 250b for side viewing element 116b. Also on the sidewall 362 of tip cover 300, on the opposing side of first side optical lens assembly 256b, is a second optical lens assembly for a second side viewing element, which may be similar to side optical lens assembly 256b and optical windows 252a and 252b of illuminators 250a and 250b for side viewing element 116b. The first side optical lens assembly 256b may provide a focal length in the range of about 3 to 100 millimeters.

An optical axis of the first side viewing element 116b may be essentially directed perpendicular to the long dimension of the endoscope. An optical axis of the second side viewing element may be essentially directed perpendicular to the long dimension of the endoscope. However, since each side viewing element typically comprises a wide angle camera, its field of view may include viewing directions at large angles to its optical axis. In accordance with some embodiments, each side viewing element has a field of view of 90 degrees or more, 120 degrees or more or up to essentially 180 degrees.

In addition, side injector opening 266 of side injector channel 666 may be located at distal end of sidewall 362. A nozzle cover 267 may be configured to fit side injector opening 266.

Additionally, nozzle cover 267 may include a nozzle 268 which may be aimed at side optical lens assembly 256b and configured for injecting fluid to wash contaminants such as blood, feces and other debris from a surface of side optical assembly 256b of side viewing element 116b. The fluid may include gas which may be used for inflating a body cavity. Optionally, nozzle 268 may be configured for cleaning both side optical lens assembly 256b and optical windows 252a and/or 252b.

According to some embodiments, side injector channel 666 may be configured to supply fluids for cleaning any of the tip elements (such as any optical assembly, optical lens assembly, windows, illuminators, and other elements).

Optionally, injector channel 646 and side injector channel 666 may be fed from same channel.

It is noted that according to some embodiments, although tip section 200 is presented herein showing one side thereof, the opposing side may include elements similar to the side elements described herein (for example, side viewing element, side optical lens assembly, injector(s), nozzle(s), illuminator(s), window(s), opening(s) and other elements).

Sidewall 362 may have a form of an essentially flat surface which assists in directing the cleaning fluid injected from injector channel 666 towards side optical lens assembly 256b and optical windows 252a and/or 252b. Lack of such flat surface may result in dripping of the cleaning fluid along the curved surface of tip section 200 of the endoscope without performing the desired cleaning action.

In accordance with an embodiment, the sidewall 362 is located in a notch/depression in the tip cover 300. This way, side injector opening 266 and corresponding side nozzle 268 may be elevated from the depressed sidewall 362 but still not significantly protrude from the level of cylindrical surface of the tip cover 300. According to an aspect of one embodiment, as shown in Figure 59C, the sidewall 362 is located in a sufficiently well-defined or deep notch/depression 5963 in the tip cover 300 such that the lens assembly of side optical lens assembly 256b stays sufficiently embedded in the notch/depression 363 and well below the level 5900 of the cylindrical surface of the tip cover 300. The notch/depression 5963 protects the sidewall 362 and components thereof (side optical lens assembly 256b, side illuminators 250a, 250b and side nozzle 268) from both longitudinal and latitudinal mechanical shocks.

It is noted that according to some embodiments, tip section 200 may include more than one side looking camera. In this case, the side looking cameras may be installed such that their fields of view are substantially opposing. However, different configurations and number of side looking cameras are possible within the general scope of the current specification.

Fluid channeling component 600 includes a side service channel 650 having a side service channel opening 350.

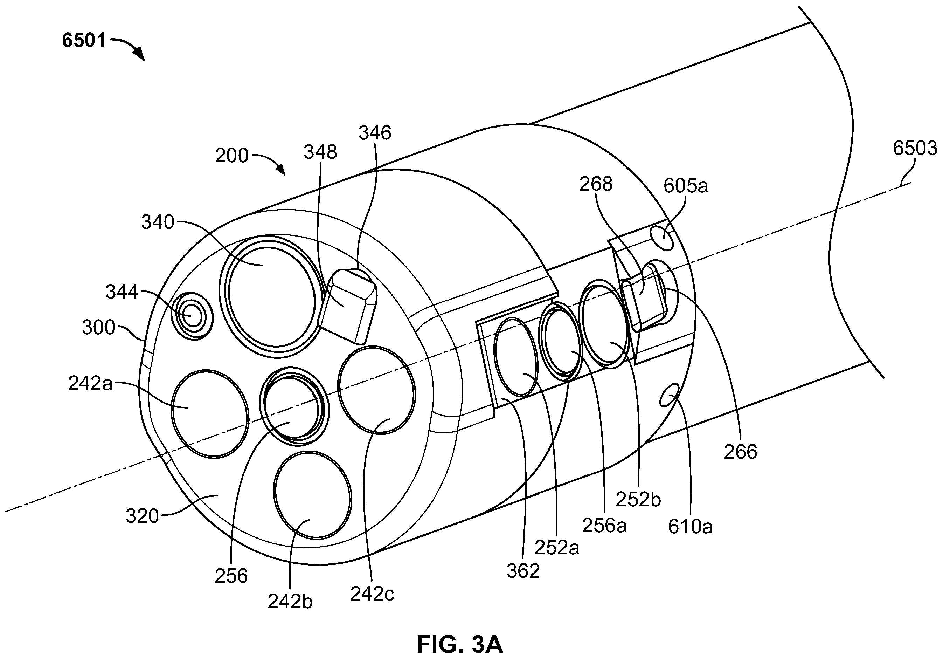

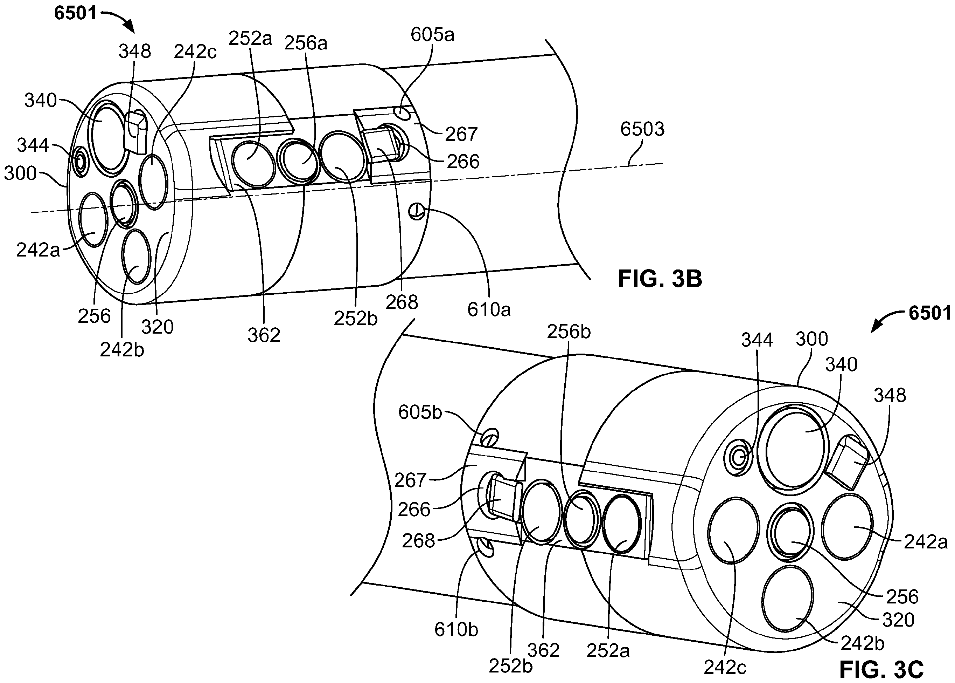

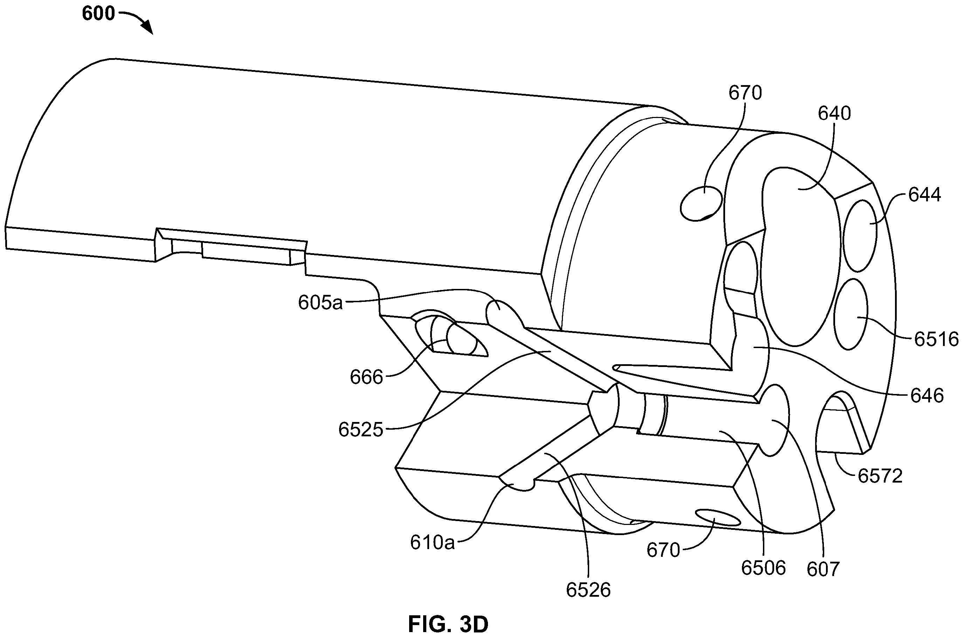

Reference is now made to Figure 1, along with Figures 3A, 3B, 3C, and 3D which show a perspective view of a tip section 200 of a multi-jet endoscope assembly 6501 comprising a plurality of side jets, in addition to a front jet, to enable improved flushing according to an embodiment of the present specification.

Tip cover 300 fits over the inner parts of the tip section 200 including electronic circuit board assembly 400 (shown in Figure 1) and fluid channeling component 600 (shown in Figure 3D) and to provide protection to the internal components in the inner parts. Holes 670 for pins for tip cover 300 are provided on fluid channeling component 600, as shown in Figure 3D. Further, Figure 3D shows a groove 6572 for an electrical cable. Tip cover 300 includes a front panel 320 having a transparent surface, window, or opening for front optical lens assembly 256, of front looking camera or viewing element 116, along with optical windows 242a, 242b and 242c of illuminators 240a, 240b and 240c, respectively.

The front panel 320 includes a working channel opening 340 of a working channel 640 and jet channel opening 344 of jet channel 644. Jet channel 644 is configured for providing a high-pressure jet of fluid, such as water or saline, for cleaning the walls of the body cavity. Also located on front panel 320 of tip cover 300 is injector opening 346 of injector channel 646 having a nozzle 348 aimed at front optical lens assembly 256. Injector channel 646 is configured for injecting fluid (liquid and/or gas) to wash contaminants such as blood, feces and other debris from a surface of front optical lens assembly 256 of front looking camera or viewing element 116. Optionally, injector channel 646 may be configured for cleaning at least a surface of front optical lens assembly 256 and one, two, or all of optical windows 242a, 242b and 242c. Injector channel 646 is fed by fluid such as water and/or gas which may be used for cleaning and/or inflating a body cavity. In one embodiment, the optical axis of the front looking camera or viewing element 116 is essentially directed along the central longitudinal axis 6503 that runs through the long dimension of the tip of the endoscope 6501.

Figure 3B shows sidewall 362 of tip cover 300 comprising a transparent surface, window, or opening to side optical lens assembly 256a for a side looking viewing element, which may be similar to front optical lens assembly 256, and optical windows 252a and 252b of illuminators for the side looking viewing element. Also, as shown in Figure 3C, the sidewall 362 of tip cover 300 on the opposing side to side optical lens assembly 256a is an optical lens assembly 256b for side looking viewing element 116b, and optical windows 252a and 252b of corresponding illuminators for side looking viewing element 116b. In one embodiment, the optical axis of one or both of the side looking viewing elements or cameras are essentially perpendicular to the optical axis (which is along the central longitudinal axis 6503 of the endoscope) of the front looking viewing element 116. In one embodiment, the optical axis of one or both of the side looking viewing elements forms an obtuse angle with the optical axis of the front viewing element 116 while in an alternate embodiment, the optical axis of one or both of the side viewing elements forms an acute angle with the optical axis of the front viewing element 116.

In addition, side injector openings 266 of corresponding side injector channels 666 are located at respective distal ends of the opposing sidewalls 362 as shown in Figures 3B and 3C. Nozzle covers 267 may be configured to fit the corresponding side injector openings 266. The nozzle covers include nozzles 268 that are aimed at side optical lens assemblies 256a, 256b and configured for injecting fluid to wash contaminants such as blood, feces and other debris from at least a surface of side optical lens assemblies 256a, 256b of the side looking viewing elements. The fluid may include gas which may be used for inflating a body cavity. Optionally, nozzles 268 may be configured for cleaning the side optical lens assembly and both optical windows on the opposing sides of the tip 200.

According to some embodiments, side injector channels 666 may be configured to supply fluids for cleaning any of the tip elements (such as any optical assembly, optical lens assembly, windows, illuminators, and other elements). Optionally, injector channel 646 and side injector channels 666 may be fed from the same channel.

As shown in Figures 3A through 3D, in accordance with an embodiment, two side jet openings 605a, 610a, fed by a common side jet channel 6506, are provided around the side periphery at the proximal end of the tip 200. Thus, the two side jet openings 605a, 610a which are fed by common side jet channel 6506 form a Y-shaped fluid conduit, described in greater detail below. The manifold shown in FIG. 3D includes a housing having a partially cylindrical shape with a curved top surface, a partially curved first side and a partially curved second side, wherein manifold housing is formed from a base portion with a first width, a first length, and a proximal surface and an elongated portion, which is attached to the base portion, with a second width, a second length, and a distal surface, wherein the first width is greater than the second width and the first length is less than the second length. A first channel 640 extends from the base portion through the elongated portion, wherein the first channel 640 has an entrance port positioned on said proximal surface of the base portion and an exit port positioned on a distal surface of the elongated portion. A second channel 644 extends from the base portion through the elongated portion, wherein the second channel 644 has an entrance port positioned on said proximal surface of the base portion and an exit port positioned on a distal surface of the elongated portion.

The Y-shaped fluid conduit comprises a central stem portion or common side jet channel 6506, a first prong portion 6525, and a second prong portion 6526, wherein the central stem portion 6506 extends from an entrance port 607 on the proximal surface of the base portion through the base portion, wherein the first prong portion 6525 extends from an end of the central portion through the base portion to an exit port on the partially curved first side; and wherein the second prong portion 6526 extends from an end of the central portion through the base portion to an exit port on the partially curved second side. In one embodiment, the exit port extending from the first prong portion 6525 forms side jet opening 605a while the exit port extending from the second prong portion 6526 forms side jet opening 610a.

A third channel 646 extends from an entrance port on the proximal surface of the base portion through to an exit port on the partially curved first side. A fourth channel 6516 extends from an entrance port on the proximal surface of the base portion through to an exit port on the partially curved second side. Each of the first, second, third, and fourth channels are fluidically isolated and separated from each other.

The common side jet channel 6506 has an entry port 607 at a proximal end of the fluid channeling component 600. Similarly, two side jet openings 605b, 610b, fed by another common side jet channel, are provided on the opposite side of side jet openings 605a and 610a. In one embodiment the two side jet openings 605a, 605b, 610a, 610b on either side of the tip are positioned in such a way that the side injector openings 266 (one on both sides of the tip) are situated between them. Additionally, in one embodiment, the two side jet openings 605a, 605b, 610a, 610b on either side of the tip are positioned close to the side optical lens assemblies 256a, 256b of the side looking cameras (on both sides of the tip) such that when fluid is ejected from the side jet openings it is propelled at an approximately 45 degree angle and past the cameras, so that a physician can see the fluid being expelled. The fluid can be water or saline.

It should be noted that, in alternate embodiments, side jet openings can be configured around the side periphery in any suitable number, including 2, 4, 6, or 8. Also, the side jet openings can have a plurality of angular configurations causing fluid to exit at different angles relative to a lateral plane that includes the side optical lens assemblies of side viewing elements and the optical windows of the corresponding illuminators but not the front optical lens assembly of the front viewing element. In one embodiment, the optical axis of the side viewing elements is perpendicular to the lateral plane as well as the optical axis of the front viewing element which is along the central longitudinal axis 6503 of the endoscope. These angles of fluid exit can range from 45 to 60 degrees or 120 to 135 degrees relative to the lateral plane. Acute angles of exit of 45 to 60 degrees enable fluid to be expelled in the direction of movement of the endoscope while obtuse angles of exit of 120 to 135 degrees enable fluid to be expelled in the direction opposite to the direction of movement of the endoscope, thereby aiding the endoscope movement within a body cavity. This is because, if the jet is directed in an opposite direction of movement of the endoscope, the resistance of the colon walls may push the scope forward like a jet engine.

In accordance with one embodiment, the side jet openings are positioned at a distance ranging from 5 to 10 millimeters, and preferably 8.5 to 9.5 millimeters from the side optical lens assemblies on the circumference of the endoscope such that the fluid exiting the openings form angles ranging from 50 degrees to 60 degrees relative to a lateral plane containing the side optical lens assemblies and corresponding side optical windows (but not containing front optical lens assembly of the front viewing element). Also, the side jet openings have a diameter of about 1.4 to 1.7 millimeters, in one embodiment.

Referring now to Figure 1 and Figures 3A through 3D, in an embodiment, a jet distributor is provided to supply fluids to each of the side jet openings, such as 605a, 605b, 610a, 610b in the multi-jet endoscope tip 6501 of Figures 3A through 3D, and the front jet 344. The jet distributor typically comprises three fluid channels to provide fluid to the front jet 344, right-side-jets 605a, 610a and left-side-jets 605b, 610b in the endoscope tip 6501.

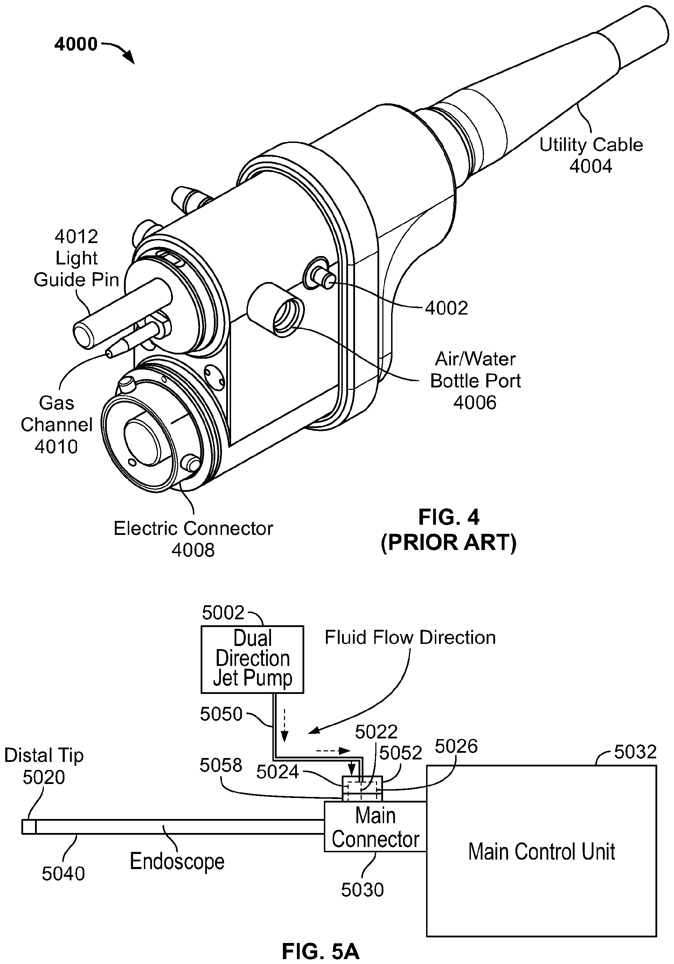

Figure 4 illustrates an exemplary embodiment of a main connector 4000, which is known in the prior art. A jet connector port 4002 is adapted for use with an endoscope that includes multiple jets. In embodiments, jet connector port (also referred to as an auxiliary water supply port) 4002 has more than one channel opening to enable jet flow to a front jet and at least one side jet of the multi-jet endoscope. The jet channels are located within a utility cable 4004 and are used to channel fluids there through and towards the respective front and side jet openings in the distal tip of the multi-jet endoscope. In embodiments, a water bottle port (a water connector) 4006, is attached to a water supply, such as a water bottle or hospital facilities, to provide fluid to an insufflation and/or irrigation system placed within the endoscope tip. Figure 4 illustrates other standard components of main connector 4000 that are provided, including an electric connector 4008 to connect the system to a source of electricity and therefore supply power to operate electric and electronic components of main connector 4000. A gas channel 4010 may provide gas flow to the tip of the endoscope. An elongated protruding member 4012 is also shown, which may be adapted to fit into a receiving structure present on the surface of a main control unit.

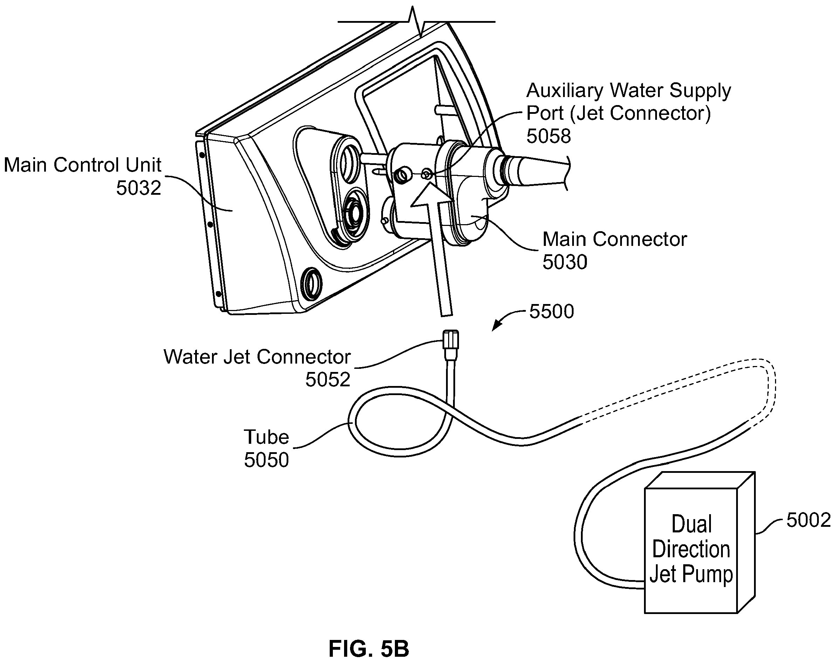

Referring to Figures 5A and 5B, in an embodiment, a dual direction jet pump 5002 is located external to an endoscope 5040 and is connected to a main control unit 5032 of endoscope 5040. In an embodiment, pump 5002 is connected to a main connector 5030 at a jet connector port 5058. In an embodiment, jet connector port 5058 is similar to jet connector port 4002 described in context of Figure 4. Figure 5A illustrates an embodiment where dual-direction jet pump 5002 enables control of fluid flow through a tube 5050 to main connector 5030. In embodiments, the single fluid flow-path enables fluid to travel through multiple fluid channels including a front-jet channel and/or side-jet channel for dispersion through a front jet opening and/or side jet opening that is located on the front panel and the side panel of a distal tip 5020 of endoscope 5040, respectively. In embodiments, a first combination of fluid channels and a second combination of fluid channels are co-linearly placed within tube 5050. In an embodiment, the first combination of fluid channels is connected to the front-jet channel within the endoscope, which leads to the front-jet opening. Similarly, the second combination of fluid channels is connected to the front-jet channel as well as the side-jet channels within the endoscope, which lead to the front-jet opening and the one or more side-jet openings.

As illustrated, in an embodiment, dual-direction jet pump 5002 supplies fluid to three jet openings in jet connector port 5058 of endoscope 5040 via at least three exiting pipelines 5022, 5024, and 5026, of which pipelines 5024 and 5026 connect to side jet channels for fluid dispersion through two side jet openings located on the side-walls of distal tip 5020. Pipeline 5022 connects to a front jet channel for fluid dispersion through a front jet opening on the front face of distal tip 5020. Hence, in the embodiment illustrated in Figure 5A, a single pump 5002 enables controlled flow of fluid through either a front jet or the front jet and the side jets.

Dual direction jet pump 5002 may connect to main control unit 5032 at port 5058, by using a coupling system. Figure 5B illustrates an exemplary coupling system 5500, in accordance with an embodiment of the present specification. In accordance with an embodiment of the present specification, coupling system 5500 comprises a water jet connector 5052, which provides a coupling mechanism between port 5058 and tube 5050. Tube 5050 connects to water jet connector 5052 and dual direction jet pump 5002. In one embodiment, connector 5052 is a luer connector, or any other type of connector that enables connecting system of small-scale fluid fittings used for making leak-free connections between a male-taper fitting and its mating female part on medical instruments.

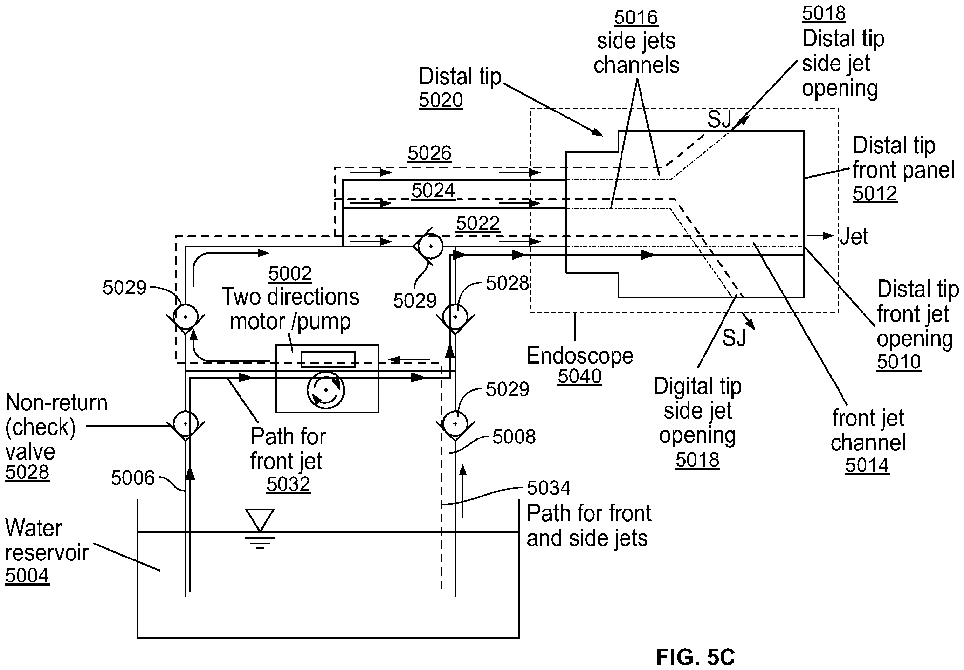

Figure 5C is a block diagram illustrating a connection between a dual-direction jet pump and an endoscope 5040, in accordance with an embodiment of the present specification. In embodiments, dual-direction jet pump includes a pump, such as jet pump 5002. Jet pump 5002 pumps fluid from a fluid source such as a water reservoir 5004, via at least two fluid pipelines 5006 and 5008. In an embodiment, pipelines 5006 and 5008 are adjacently placed within single tube, conduit or pipeline 5050. In embodiments, pipeline 5006 forms a part of the first combination of fluid channels that supply fluid to front jet opening 5010, and pipeline 5008 forms a part of the second combination of fluid channels that supply fluid to front jet opening 5010 and side jet openings 5018.

In embodiments, a first fluid pipeline 5006 provides a path for pumping water for a front jet through a front jet channel 5014 and via a front jet opening 5010 on a front panel 5012 of a distal tip 5020 of endoscope 5040. Water pumped by pump 5002 through first fluid pipeline 5006 may be directed through a front jet channel 5014 to be dispersed from front jet opening 5010. Figure 5C illustrates a fluid path 5032 corresponding to the first combination of fluid channels, for fluid to flow towards front jet opening 5010. Path 5032 is shown in the form of a thick line, through pipeline 5006, via pump 5002, through channel 5014, towards distal tip front jet opening 5010.

In embodiments, a second fluid pipeline 5008 provides a path for pumping water for a front and one or more side jets of the endoscope. Figure 5C illustrates a fluid path 5034 corresponding to the second combination of fluid channels, for fluid to flow towards front jet opening 5010 and one or more side jet openings 5018, of endoscope 5040. Path 5034 is shown in the form of a thick dashed line, through pipeline 5008, via pump 5002, towards distal tip front jet opening 5010 and side jet openings 5018. Water pumped through the second fluid pipeline 5008 may travel through front jet channel 5014 and side jet channels 5016, for dispersion through front jet opening 5010 and one or more distal tip side jet openings 5018. Jet openings 5010 and 5018 are located within distal tip 5020 of the endoscope.

Pump 5002 is a dual-direction pump adapted to direct the fluid jet's flow to the front jet, to one or more of the side jets, or to both the front and side jets concurrently. The fluid is supplied by pump 5002 to three jet openings in tip 5020 of endoscope 5040 via three fluid pipelines 5022, 5024, and 5026. In an embodiment, each of the three exiting fluid pipelines 5022, 5024, and 5026 supply fluid to a fluid channel positioned within tip 5020. In an embodiment, pipelines 5024 and 5026 supply fluid to side jet channels 5016; and pipeline 5022 supplies fluid to front jet channel 5014.

In an embodiment, the three exiting fluid pipelines 5022, 5024, and 5026 are located within tube 5050 that includes pipelines 5006 and 5008. In an embodiment, pipeline 5008, which provides the path for fluid to travel to front and side jet openings 5010 and 5018, is connected to at least two or more pipelines that branch out from pipeline 5008. In the figure, pipeline 5008 is shown to branch out in to pipelines 5022, 5024, and 5026. In embodiments, pipelines 5022, 5024, and 5026 are parallel pipelines. Pipeline 5022 is connected to front jet channel 5014, and pipelines 5024 and 5026 are connected to side jet channels 5016. In embodiments, pipeline 5006 also merges with pipeline 5022, in order to connect subsequently with front jet channel 5014. In embodiments, pipelines 5022, 5024, and 5026 connect with their corresponding channels through a main connector (shown in Figure 5B). The three pipelines 5022, 5024, and 5026 are embedded within tube 5050 (shown in Figures 5A and 5B) along connector 5052 (shown in Figures 5A and 5B). Pipelines 5022, 5024, and 5026 are aligned into the associated channels in connector port 5058 (shown in Figures 5A and 5B). The main connector is also coupled with a controller unit that acts as a main control unit for the endoscope.

In various embodiments, in order to activate the jet and wash a lumen in a patient's body, a user/physician operating endoscope 5040 may use an activation system such as, but not limited to, a button located either on a handle of the endoscope, on the main control unit, or on a control panel of the endoscope, that, when pressed, causes a controller to activate the pump, thereby causing water to flow in one of two directions or to shut off. For example, once the button on an endoscope is pressed, a controller, which is in data communication with the button, causes the dual-direction jet pump 5002 to start providing fluid at a pre-determined rate to one of, two of, or each of the three fluid channels 5022, 5024, and 5026, of the endoscope. In another embodiment, pushing the button once may activate the pump in order to apply pressure to the fluid through pipeline 5006 and supply fluid to the front jet. In this embodiment, pushing the button twice may cause a controller to activate the pump in order to apply pressure to the fluid through pipeline 5008 and supply fluid to the front jet and the side jets. In another embodiment, the user/physician may be required to step on a foot pedal to cause a controller to activate jet-pump 5002. Thus, in embodiments, two direction motor/pump 5002 is operated by a pushed button, a lever, or any other known trigger, or activation, mechanism to cause the fluid to flow through one, two, and/or all three fluid channels and pressing the activation mechanism a predetermined number of times corresponds with causing fluid to flow through a specific one or combination of the fluid channels. During operation of embodiments of the present specification, the user selects an option to either supply fluid through front jet opening 5010, or through front jet opening 5010 and side jet opening 5018.

If the user choses to supply fluid to only front jet opening 5010, pump 5002 is activated to apply pressure to fluid through pipeline 5006. In an embodiment, a series of non-return check valves 5028 are placed along path 5032, which are also activated in order to control the operation. Each check valve 5028 ensures that fluid flows through the pipeline in one direction only. Pump 5002 pumps fluid in the direction indicated by path 5032 in order to supply fluid to front jet opening 5010. A first valve 5028 may be located in pipeline 5006 between reservoir 5004 and pump 5002 that ensures the fluid flows in the desired direction of path 5032. A second check valve 5028 may be located between pump 5002 and pipeline 5022 that is connected to front jet channel 5014. Second valve 508 also ensures that fluid flows in the direction of path 5032, while pump 5002 pumps fluid from pipeline 5006 in the same direction.

Alternatively, if the user choses to supply fluid to front jet opening 5010 and side jet openings 5018 simultaneously, pump 5002 is activated to pump fluid through pipeline 5008 in a direction that is opposite to the direction for pipeline 5006. A series of non-return check valves 5029 ensure that the fluid flows in the direction of path 5034 for supplying fluid to front and side jet openings 5010, 5018. In some embodiments, a first check valve 5029 is placed between reservoir 5004 and pump 5002 along pipeline 5008. A second check valve 5029 may be placed along pipeline 5008 between pump 5002 and before the point where pipeline 5008 branches out to pipelines 5022, 5024, and 5026.

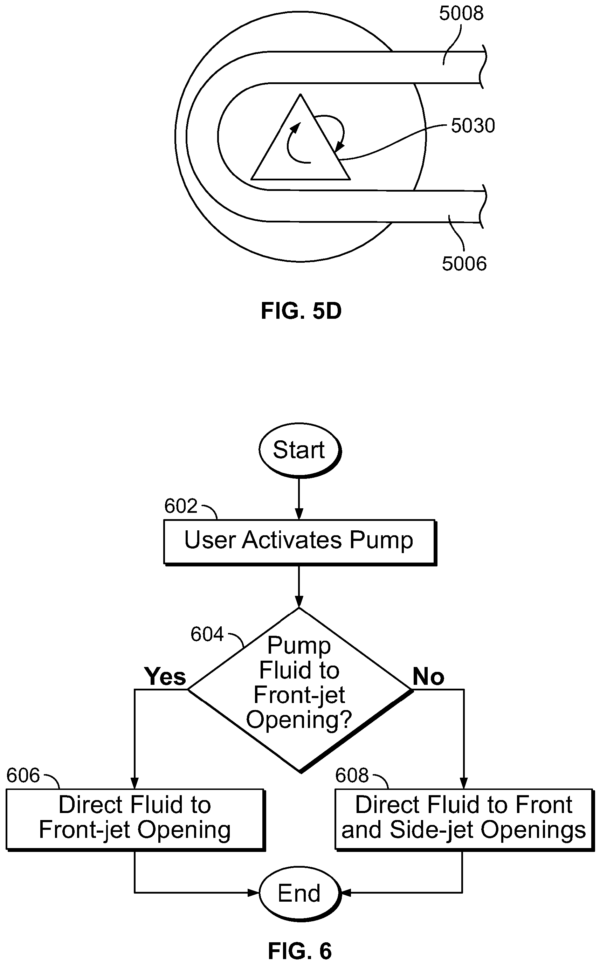

Figure 5D illustrates an exemplary operation of dual direction pump 5002, in accordance with an embodiment of the present specification. In embodiments, pump 5002 is a peristaltic pump, which operates on the basis of alternating compression and relaxation of a tube. Pipelines 5006 and 5008 may form a single continuous tube that is wound around a rotating shoe or roller 5030. Rotating shoe or roller 5030 forms pump 5002. Roller 5030 passes along the length of the tube, resulting in two ends of the tube that are on the two sides of roller 5030. These two ends may form the two paths 5032 (corresponding to pipeline 5006) and 5034 (corresponding to pipeline 5008). During operation, roller 5030 compresses the tube and creates a seal between the two sides of the tube, such that fluid is suctioned through the selected one of the two paths 5006 and 5008. Rotation of roller 5030 in one direction may pull fluid through one path, while rotation of roller 5030 in an opposite direction may pull the fluid through the other path. Therefore, operating in one direction may supply fluids to front and side jets, while operating in the other direction may supply fluids to the front jet only. In embodiments, a single trigger mechanism (single button, lever, pedal, or any other) may be toggled to change the direction of pump 5002.

Figure 6 is a flow chart illustrating an exemplary method of controlling a flow direction of fluid from a source external to an endoscope into a plurality of fluid channels positioned within the endoscope. Simultaneously referring to Figures 5A, 5B, and 5C, at step 602, the user activates pump 5002 connected to tube 5050. In embodiments, tube 5050 includes the first and second combinations of fluid channels that are collinearly placed within tube 5050. The first combination of fluid channels, corresponding to path 5032, direct fluid to front-jet opening 5010; whereas the second combinations of fluid channels that are collinearly placed within tube 5050. The first combination of fluid channels, corresponding to path 5032, direct fluid to front-jet opening 5010; whereas the second combination of fluid channels, corresponding to path 5034, direct fluid to front-jet opening 5010 and side-jet openings 5018. Therefore, at step 604, the system determines whether the user has activated the pump to direct fluid to front-jet opening 5010 only or to front-jet opening 5010 and side-jet openings 5018 simultaneously. If the user has activated the first option, at 606, the fluid is directed by pump 5002 via path 5032 comprising the first combination of fluid channels, to front-jet channel 5014. Alternatively, if the user has activated the second option, at 608, the fluid is directed by pump 5002 via path 5034 comprising the second combination of fluid channels, to front-jet channel 5014 and a side-jet channels 5016.

The above examples are merely illustrative of the many applications of the system of present invention. Although only a few embodiments of the present invention have been described herein, it should be understood that the present invention might be embodied in many other specific forms without departing from the scope of the invention. Therefore, the present examples and embodiments are to be considered as illustrative and not restrictive, and the invention may be modified within the scope of the appended claims.

## Claims

1. An apparatus for controlling a flow direction of fluid from a fluid source (5004) external to an endoscope (5040) into a plurality of fluid channels positioned within a distal end of the endoscope (5040), comprising:
a tube (5050) comprising a first combination of fluid channels (5032) and a second combination of fluid channels (5034);
a pump (5002) connected to the tube (5050), wherein the pump is adapted to direct fluid from the fluid source (5004) to at least one of the first combination of fluid channels (5032) and the second combination of fluid channels (5034) in said tube (5050);
a controller to activate the pump (5002), wherein the controller is configured to cause the pump (5002) to direct fluid to the first combination of fluid channels (5032) upon a first activation of the controller, and wherein the controller is configured to cause the pump (5002) to direct fluid to the second combination of fluid channels (5034) upon a second activation of the controller; and
an endoscope (5040);
wherein the first combination of fluid channels (5032) comprises a fluid channel (5014) that opens through a front jet (5010) in a distal tip (5020) of the endoscope (5040); and
wherein the second combination of fluid channels (5034) comprises a fluid channel (5016) that opens through at least one side jet (5018) in the distal tip (5020) of the endoscope (5040).

2. The apparatus of claim 1, wherein the pump (5002) is a peristaltic pump.

3. The apparatus of claim 1, further comprising a user trigger to control said controller, wherein the user trigger comprises a button and wherein the button is configured such that pressing the button causes the first activation of the controller and pressing the button twice causes the second activation of the controller.

4. The apparatus of claim 1, further comprising a user trigger to control said controller, wherein the user trigger comprises a lever and wherein the lever is configured such that pulling the lever causes the first activation of the controller and pushing the lever causes the second activation of the controller.

5. The apparatus of claim 1, further comprising a user trigger to control said controller, wherein the user trigger comprises a pedal and wherein the pedal is configured such that stepping on the pedal causes the first activation of the controller and stepping on the pedal twice causes the second activation of the controller.

6. The apparatus of claim 1, wherein the first combination of fluid channels (5032) and the second combination of fluid channels (5034) are co-linearly placed within the tube (5050).

7. The apparatus of claim 1, wherein the second combination of fluid channels (5034) comprises a fluid channel (5008) that opens through a front jet (5010) in the distal tip (5020) of the endoscope (5040) and the at least one side jet (5018) in the distal tip (5020) of the endoscope (5040).

8. The apparatus of claim 1 wherein the pump (5002) is configured to direct fluid in a first direction through the first combination of fluid channels (5032) upon said first activation of the controller, wherein the pump (5002) is configured to direct fluid in a second direction through the second combination of fluid channels (5034) upon said second activation of the controller, and wherein the first direction is different from the second direction.

9. The apparatus of claim 1, further comprising a first check valve (5028) in a first fluid channel (5006) positioned between said fluid source (5004) and the pump (5002) and a second check valve (5029) in a second fluid channel (5008) positioned between said fluid source (5004) and the pump (5002), wherein the first fluid channel (5006) is separate from the second fluid channel (5008), wherein optionally the first fluid channel (5006) is in fluid communication with the first combination of fluid channels (5032), wherein the second fluid channel (5008) is in fluid communication with the second combination of fluid channels (5034), wherein the first fluid channel (5006) is not in fluid communication with the second combination of fluid channels (5034), and wherein the second fluid channel (5008) is not in fluid communication with the first combination of fluid channels (5032).

10. A method for controlling a flow direction of fluid from a fluid source (5004) external to an endoscope (5040) into a plurality of fluid channels positioned within the endoscope (5040), comprising:
receiving a user input into a trigger;
based upon said trigger, using a controller to activate a pump (5002) connected to a tube (5050), wherein said tube (5050) comprises a first combination of fluid channels (5032) and a second combination of fluid channels (5034), wherein, upon a first activation of the pump (5002), said pump causes fluid to flow in a first direction from the fluid source (5004) to the first combination of fluid channels (5032), and wherein, upon a second activation of the pump (5002), said pump causes fluid to flow in a second direction from the fluid source (5004) to the second combination of fluid channels (5034);
wherein the first combination of fluid channels (5032) comprises a fluid channel (5014) that opens through a front jet (5010) in a distal tip (5020) of the endoscope (5040); and
wherein the second combination of fluid channels (5034) comprises a fluid channel (5016) that opens through at least one side jet (5018) in the distal tip (5020) of the endoscope (5040).

11. The method of claim 10, wherein said trigger comprises a button, wherein pressing the button once enables said first activation of the pump (5002), and wherein pressing the button twice enables the second activation of the pump (5002), and/or wherein said trigger comprises a lever, wherein pulling the lever enables the first activation of the pump (5002) and wherein pushing the lever enables the second activation of the pump (5002), and/or
wherein said trigger comprises a lever, wherein pulling or pushing the lever once enables the first activation of the pump (5002) and wherein pushing or pulling the lever twice enables the second activation of the pump (5002), and/or
wherein said trigger comprises a pedal, wherein pushing the pedal once causes the first activation of the pump (5002) and wherein pushing the pedal twice causes the second activation of the pump (5002).

12. The method of claim 10, wherein the first combination of fluid channels (5032) and the second combination of fluid channels (5034) are co-linearly placed within the tube (5050).

13. The method of claim 10, wherein the pump (5050) comprises a peristaltic pump, preferably wherein the pump (5002) is configured to direct fluid in a first direction through the first combination of fluid channels (5032) upon said first activation of the pump (5002), wherein the pump (5002) is configured to direct fluid in a second direction through the second combination of fluid channels (5034) upon said second activation of the pump (5002), and wherein the first direction is different from the second direction.

14. The method of claim 10, further comprising a first check valve (5028) in a first fluid channel (5006) positioned between said fluid source (5004) and the pump (5002) and a second check valve (5029) in a second fluid channel (5008) positioned between said fluid source (5004) and the pump (5002), wherein the first fluid channel (5006) is separate from the second fluid channel (5008).

15. The method of claim 14, wherein the first fluid channel (5006) is in fluid communication with the first combination of fluid channels (5032), wherein the second fluid channel (5008) is in fluid communication with the second combination of fluid channels (5034), wherein the first fluid channel (5006) is not in fluid communication with the second combination of fluid channels (5034), and wherein the second fluid channel (5008) is not in fluid communication with the first combination of fluid channels (5032).

## Patentansprüche

1. Vorrichtung zum Steuern einer Strömungsrichtung eines Fluids von einer Fluidquelle (5004) außerhalb eines Endoskops (5040) in mehrere Fluidkanäle, die innerhalb eines distalen Endes des Endoskops (5040) positioniert sind, wobei die Vorrichtung aufweist:
einen Schlauch (5050), der eine erste Kombination von Fluidkanälen (5032) und eine zweite Kombination von Fluidkanälen (5034) aufweist;
eine mit dem Schlauch (5050) verbundene Pumpe (5002), wobei die Pumpe eingerichtet ist, Fluid von der Fluidquelle (5004) zur ersten Kombination von Fluidkanälen (5032) und/oder zweiten Kombination von Fluidkanälen (5034) in dem Schlauch (5050) zu leiten;
einen Controller zum Ansteuern der Pumpe (5002), wobei der Controller eingerichtet ist, die Pumpe (5002) bei einer ersten Ansteuerung des Controllers zu veranlassen, Fluid zur ersten Kombination von Fluidkanälen (5032) zu leiten, und wobei der Controller eingerichtet ist, die Pumpe (5002) bei einer zweiten Ansteuerung des Controllers zu veranlassen, Fluid zu der zweiten Kombination von Fluidkanälen (5034) zu leiten; und
ein Endoskop (5040);
wobei die erste Kombination von Fluidkanälen (5032) einen Fluidkanal (5014) aufweist, der durch einen Vorderstrahl (5010) in einer distalen Spitze (5020) des Endoskops (5040) mündet; und
wobei die zweite Kombination von Fluidkanälen (5034) einen Fluidkanal (5016) aufweist, der durch zumindest einen Seitenstrahl (5018) in der distalen Spitze (5020) des Endoskops (5040) mündet.

2. Vorrichtung nach Anspruch 1, wobei die Pumpe (5002) eine Peristaltikpumpe ist.

3. Vorrichtung nach Anspruch 1, ferner aufweisend einen Nutzerauslöser, um den Controller zu steuern, wobei der Nutzerauslöser einen Knopf aufweist und wobei der Knopf derart eingerichtet ist, dass das Drücken des Knopfes die erste Ansteuerung des Controllers bewirkt und das zweimalige Drücken des Knopfes die zweite Ansteuerung des Controllers bewirkt.

4. Vorrichtung nach Anspruch 1, ferner aufweisend einen Nutzerauslöser, um den Controller zu steuern, wobei der Nutzerauslöser einen Hebel aufweist und wobei der Hebel derart eingerichtet ist, dass das Ziehen des Hebels die erste Ansteuerung des Controllers bewirkt und das Drücken des Hebels die zweite Ansteuerung des Controllers bewirkt.

5. Vorrichtung nach Anspruch 1, ferner aufweisend einen Nutzerauslöser, um den Controller zu steuern, wobei der Nutzerauslöser ein Pedal aufweist und wobei das Pedal derart eingerichtet ist, dass das Betätigen des Pedals die erste Ansteuerung des Controllers bewirkt und das zweimalige Betätigen des Pedals die zweite Ansteuerung des Controllers bewirkt.

6. Vorrichtung nach Anspruch 1, wobei die erste Kombination von Fluidkanälen (5032) und die zweite Kombination von Fluidkanälen (5034) innerhalb des Schlauchs (5050) kollinear platziert sind.

7. Vorrichtung nach Anspruch 1, wobei die zweite Kombination von Fluidkanälen (5034) einen Fluidkanal (5008) aufweist, der durch einen Vorderstrahl (5010) in der distalen Spitze (5020) des Endoskops (5040) und den zumindest einen Seitenstrahl (5018) in der distalen Spitze (5020) des Endoskops (5040) mündet.

8. Vorrichtung nach Anspruch 1, wobei die Pumpe (5002) bei der ersten Ansteuerung des Controllers eingerichtet ist, Fluid in einer ersten Richtung durch die erste Kombination von Fluidkanälen (5032) zu leiten, wobei die Pumpe (5002) bei der zweiten Ansteuerung des Controllers eingerichtet ist, Fluid in einer zweiten Richtung durch die zweite Kombination von Fluidkanälen (5034) zu leiten, und wobei sich die erste Richtung von der zweiten Richtung unterscheidet.

9. Vorrichtung nach Anspruch 1, ferner aufweisend ein erstes Rückschlagventil (5028) in einem ersten Fluidkanal (5006), das zwischen der Fluidquelle (5004) und der Pumpe (5002) positioniert ist, und ein zweites Rückschlagventil (5029) in einem zweiten Fluidkanal (5008), das zwischen der Fluidquelle (5004) und der Pumpe (5002) positioniert ist, wobei der erste Fluidkanal (5006) vom zweiten Fluidkanal (5008) getrennt ist, wobei der erste Fluidkanal (5006) optional in fluidischer Kommunikation mit der ersten Kombination von Fluidkanälen (5032) steht, wobei der zweite Fluidkanal (5008) in fluidischer Kommunikation mit der zweiten Kombination von Fluidkanälen (5034) steht, wobei der erste Fluidkanal (5006) nicht in fluidischer Kommunikation mit der zweiten Kombination von Fluidkanälen (5034) steht, und wobei der zweite Fluidkanal (5008) nicht in fluidischer Kommunikation mit der ersten Kombination von Fluidkanälen (5032) steht.

10. Verfahren zum Steuern einer Strömungsrichtung eines Fluid von einer Fluidquelle (5004) außerhalb eines Endoskops (5040) in mehrere Fluidkanäle, die innerhalb des Endoskops (5040) positioniert sind, wobei das Verfahren aufweist:
Empfang einer Nutzereingabe in einen Auslöser;
basierend auf dem Auslöser, Verwenden eines Controllers, um eine Pumpe (5002) anzusteuern, die mit einem Schlauch (5050) verbunden ist, wobei der Schlauch (5050) eine erste Kombination von Fluidkanälen (5032) und eine zweite Kombination von Fluidkanälen (5034) aufweist, wobei, bei einer ersten Ansteuerung der Pumpe (5002), die Pumpe bewirkt, dass Fluid in einer ersten Richtung von der Fluidquelle (5004) zu der ersten Kombination von Fluidkanälen (5032) strömt, und wobei, bei einer zweiten Ansteuerung der Pumpe (5002), die Pumpe bewirkt, dass Fluid in einer zweiten Richtung von der Fluidquelle (5004) zu der zweiten Kombination von Fluidkanälen (5034) strömt;
wobei die erste Kombination von Fluidkanälen (5032) einen Fluidkanal (5014) aufweist, der durch einen Vorderstrahl (5010) in einer distalen Spitze (5020) des Endoskops (5040) mündet; und
wobei die zweite Kombination von Fluidkanälen (5034) einen Fluidkanal (5016) aufweist, der durch zumindest einen Seitenstrahl (5018) in der distalen Spitze (5020) des Endoskops (5040) mündet.

11. Verfahren nach Anspruch 10, wobei der Auslöser einen Knopf aufweist, wobei das einmalige Drücken des Knopfes die erste Ansteuerung der Pumpe (5002) veranlasst und wobei das zweimalige Drücken des Knopfes die zweite Ansteuerung der Pumpe (5002) veranlasst, und/oder wobei der Auslöser einen Hebel aufweist, wobei das Ziehen des Hebels die erste Ansteuerung der Pumpe (5002) veranlasst und wobei das Drücken des Hebels die zweite Ansteuerung der Pumpe (5002) veranlasst, und/oder
wobei der Auslöser einen Hebel aufweist, wobei das einmalige Drücken oder Ziehen des Hebels die erste Ansteuerung der Pumpe (5002) veranlasst und wobei das zweimalige Drücken oder Ziehen des Hebels die zweite Ansteuerung der Pumpe (5002) veranlasst, und/oder
wobei der Auslöser ein Pedal aufweist, wobei das einmalige Drücken des Pedals die erste Ansteuerung der Pumpe (5002) veranlasst und wobei das zweimalige Drücken des Pedals die zweite Ansteuerung der Pumpe (5002) veranlasst.

12. Verfahren nach Anspruch 10, wobei die erste Kombination von Fluidkanälen (5032) und die zweite Kombination von Fluidkanälen (5034) kollinear innerhalb des Schlauchs (5050) platziert sind.

13. Verfahren nach Anspruch 10, wobei die Pumpe (5050) eine Peristaltikpumpe aufweist, wobei die Pumpe (5002) bevorzugt eingerichtet ist, Fluid bei der ersten Ansteuerung der Pumpe (5002) in einer ersten Richtung durch die erste Kombination von Fluidkanälen (5032) zu leiten, wobei die Pumpe (5002) eingerichtet ist, Fluid bei der zweiten Ansteuerung der Pumpe (5002) in einer zweiten Richtung durch die zweite Kombination von Fluidkanälen (5034) zu leiten, und wobei sich die erste Richtung von der zweiten Richtung unterscheidet.

14. Verfahren nach Anspruch 10, ferner aufweisend ein erstes Rückschlagventil (5028) in einem ersten Fluidkanal (5006), das zwischen der Fluidquelle (5004) und der Pumpe (5002) positioniert ist, und ein zweites Rückschlagventil (5029) in einem zweiten Fluidkanal (5008), das zwischen der Fluidquelle (5004) und der Pumpe (5002) positioniert ist, wobei der erste Fluidkanal (5006) vom zweiten Fluidkanal (5008) getrennt ist.

15. Verfahren nach Anspruch 14, wobei der erste Fluidkanal (5006) in fluidischer Kommunikation mit der ersten Kombination von Fluidkanälen (5032) steht, wobei der zweite Fluidkanal (5008) in fluidischer Kommunikation mit der zweiten Kombination von Fluidkanälen (5034) steht, wobei der erste Fluidkanal (5006) nicht in fluidischer Kommunikation mit der zweiten Kombination von Fluidkanälen (5034) steht, und wobei der zweite Fluidkanal (5008) nicht in fluidischer Kommunikation mit der ersten Kombination von Fluidkanälen (5032) steht.

## Revendications

1. Appareil pour commander une direction d'écoulement de fluide d'une source de fluide (5004) extérieure à un endoscope (5040) dans une pluralité de canaux à fluide positionnés à l'intérieur d'une extrémité distale de l'endoscope (5040), comprenant:
un tube (5050) comprenant une première combinaison de canaux à fluide (5032) et une seconde combinaison de canaux à fluide (5034);
une pompe (5002) connectée au tube (5050), où la pompe est adaptée pour diriger du fluide de la source de fluide (5004) à au moins l'une de la première combinaison de canaux à fluide (5032) et de la seconde combinaison de canaux à fluide (5034) dans ledit tube (5050);
un organe de commande pour activer la pompe (5002), où l'organe de commande est configuré pour amener la pompe (5002) à diriger du fluide jusqu'à la première combinaison de canaux à fluide (5032) lors d'une première activation de l'organe de commande, et où l'organe de commande est configuré pour amener la pompe (5002) à diriger du fluide jusqu'à la seconde combinaison de canaux à fluide (5034) lors d'une seconde activation de l'organe de commande; et
un endoscope (5040);
où la première combinaison de canaux à fluide (5032) comprend un canal à fluide (5014) qui débouche par un jet frontal (5010) dans une pointe distale (5020) de l'endoscope (5040); et
où la seconde combinaison de canaux à fluide (5034) comprend un canal à fluide (5016) qui débouche par au moins un jet latéral (5018) dans la pointe distale (5020) de l'endoscope (5040).

2. Appareil selon la revendication 1, où la pompe (5002) est une pompe péristaltique.

3. Appareil selon la revendication 1, comprenant en outre un déclencheur utilisateur pour commander ledit organe de commande, où le déclencheur utilisateur comprend un bouton et où le bouton est configuré de telle sorte que le fait de presser le bouton provoque la première activation de l'organe de commande et le fait de presser le bouton deux fois provoque la seconde activation de l'organe de commande.

4. Appareil selon la revendication 1, comprenant en outre un déclencheur utilisateur pour commander ledit organe de commande, où le déclencheur utilisateur comprend un levier et où le levier est configuré de telle sorte que le fait de tirer le levier provoque la première activation de l'organe de commande et le fait de pousser le levier provoque la seconde activation de l'organe de commande.

5. Appareil selon la revendication 1, comprenant en outre un déclencheur utilisateur pour commander ledit organe de commande, où le déclencheur utilisateur comprend une pédale et où la pédale est configurée de telle sorte que le fait d'appuyer sur la pédale provoque la première activation de l'organe de commande et le fait d'appuyer deux fois sur la pédale provoque la seconde activation de l'organe de commande.

6. Appareil selon la revendication 1, où la première combinaison de canaux à fluide (5032) et la seconde combinaison de canaux à fluide (5034) sont placées de manière colinéaire dans le tube (5050).

7. Appareil selon la revendication 1, où la seconde combinaison de canaux à fluide (5034) comprend un canal à fluide (5008) qui débouche par un jet frontal (5010) dans la pointe distale (5020) de l'endoscope (5040) et le au moins un jet latéral (5018) dans la pointe distale (5020) de l'endoscope (5040).

8. Appareil selon la revendication 1 où la pompe (5002) est configurée pour diriger du fluide dans une première direction par la première combinaison de canaux à fluide (5032) lors de ladite première activation de l'organe de commande, où la pompe (5002) est configurée pour diriger du fluide dans une seconde direction par la seconde combinaison de canaux à fluide (5034) lors de ladite seconde activation de l'organe de commande, et où la première direction est différente de la seconde direction.

9. Appareil selon la revendication 1, comprenant en outre un premier clapet anti-retour (5028) dans un premier canal à fluide (5006) positionné entre ladite source de fluide (5004) et la pompe (5002) et un second clapet anti-retour (5029) dans un second canal à fluide (5008) positionné entre ladite source de fluide (5004) et la pompe (5002), où le premier canal à fluide (5006) est séparé du second canal à fluide (5008), où éventuellement le premier canal à fluide (5006) est en communication fluidique avec la première combinaison de canaux à fluide (5032), où le second canal à fluide (5008) est en communication fluidique avec la seconde combinaison de canaux à fluide (5034), où le premier canal à fluide (5006) n'est pas en communication fluidique avec la seconde combinaison de canaux à fluide (5034), et où le second canal à fluide (5008) n'est pas en communication fluidique avec la première combinaison de canaux à fluide (5032).

10. Procédé pour commander une direction d'écoulement de fluide d'une source de fluide (5004) extérieure à un endoscope (5040) dans une pluralité de canaux à fluide positionnés à l'intérieur de l'endoscope (5040), comprenant:
la réception d'une entrée d'utilisateur dans un déclencheur;
sur la base dudit déclencheur, l'utilisation d'un organe de commande pour activer une pompe (5002) connectée à un tube (5050), où ledit tube (5050) comprend une première combinaison de canaux à fluide (5032) et une seconde combinaison de canaux à fluide (5034), où, lors d'une première activation de la pompe (5002), ladite pompe amène du fluide à s'écouler dans une première direction depuis la source de fluide (5004) jusqu'à la première combinaison de canaux à fluide (5032), et où, lors d'une seconde activation de la pompe (5002), ladite pompe amène du fluide à s'écouler dans une seconde direction depuis la source de fluide (5004) jusqu'à la seconde combinaison de canaux à fluide (5034);
où la première combinaison de canaux à fluide (5032) comprend un canal à fluide (5014) qui débouche par un jet frontal (5010) dans une pointe distale (5020) de l'endoscope (5040); et
où la seconde combinaison de canaux à fluide (5034) comprend un canal à fluide (5016) qui débouche par au moins un jet latéral (5018) dans la pointe distale (5020) de l'endoscope (5040).

11. Procédé selon la revendication 10, où ledit déclencheur comprend un bouton, où le fait de presser le bouton une fois permet ladite première activation de la pompe (5002), et où le fait de presser le bouton deux fois permet la seconde activation de la pompe (5002), et/ou
où ledit déclencheur comprend un levier, où le fait de tirer le levier permet la première activation de la pompe (5002) et où le fait de pousser le levier permet la seconde activation de la pompe (5002), et/ou
où ledit déclencheur comprend un levier, où le fait de tirer ou de pousser le levier une fois permet la première activation de la pompe (5002) et où le fait de pousser ou de tirer le levier deux fois permet la seconde activation de la pompe (5002), et/ou
où ledit déclencheur comprend une pédale, où le fait de pousser la pédale une fois provoque la première activation de la pompe (5002) et où le fait de pousser la pédale deux fois provoque la seconde activation de la pompe (5002).

12. Procédé selon la revendication 10, où la première combinaison de canaux à fluide (5032) et la seconde combinaison de canaux à fluide (5034) sont placées de manière colinéaire à l'intérieur du tube (5050).

13. Procédé selon la revendication 10, où la pompe (5050) comprend une pompe péristaltique, de préférence où la pompe (5002) est configurée pour diriger du fluide dans une première direction par la première combinaison de canaux à fluide (5032) lors de ladite première activation de la pompe (5002), où la pompe (5002) est configurée pour diriger du fluide dans une seconde direction par la seconde combinaison de canaux à fluide (5034) lors de ladite seconde activation de la pompe (5002), et où la première direction est différente de la seconde direction.

14. Procédé selon la revendication 10, comprenant en outre un premier clapet anti-retour (5028) dans un premier canal à fluide (5006) positionné entre ladite source de fluide (5004) et la pompe (5002) et un second clapet anti-retour (5029) dans un second canal à fluide (5008) positionné entre ladite source de fluide (5004) et la pompe (5002), où le premier canal à fluide (5006) est séparé du second canal à fluide (5008).

15. Procédé selon la revendication 14, où le premier canal à fluide (5006) est en communication fluidique avec la première combinaison de canaux à fluide (5032), où le second canal à fluide (5008) est en communication fluidique avec la seconde combinaison de canaux à fluide (5034), où le premier canal à fluide (5006) n'est pas en communication fluidique avec la seconde combinaison de canaux à fluide (5034), et où le second canal à fluide (5008) n'est pas en communication fluidique avec la première combinaison de canaux à fluide (5032).
